# EUROPEAN PATENT SPECIFICATION

(11) **EP 2 514 351 B1**
(45) Date of publication and mention of the grant of the patent: **12.04.2017**
(21) Application number: 10837373.9
(22) Date of filing: 04.11.2010
(51) Int. Cl.: A61B 1/00, A61B 5/07

(54) **CONTROL SIGNAL TRANSMITTING APPARATUS**
VORRICHTUNG ZUR ÜBERTRAGUNG VON STEUERSIGNALEN
APPAREIL DE TRANSMISSION DE SIGNAL DE COMMANDE

(30) Priority: 18.12.2009 JP 2009287719
(43) Date of publication of application: 24.10.2012
(73) Proprietor: Olympus Corporation, Tokyo 192-8507 (JP)
(72) Inventor: SATO Ken, Tokyo 151-0072 (JP); SAKAI Youhei, Shibuya-ku Tokyo 151-0072 (JP); YOSHIZAWA Fukashi, Shibuya-ku Tokyo 151-0072 (JP)
(74) Representative: Winter, Brandl, Fürniss, Hübner, Röss, Kaiser, Polte - Partnerschaft mbB
(86) International application number: PCT/JP2010/069607
(87) International publication number: WO 2011/074344

(56) References cited:
- EP-A1- 1 834 568
- EP-A1- 1 967 127
- JP-A- 2006 187 424
- JP-A- 2007 089 893
- JP-A- 2009 089 907
- JP-A- 2009 516 562
- US-A1- 2007 129 602
- US-A1- 2008 060 952

## Description

### Technical Field

The present invention relates to a control signal transmitting apparatus that transmits a control signal for performing control of start and stop to a living body information acquiring apparatus that is introduced into an inside of a subject and acquires information in the subject.

### Background Art

An endoscope is used for, for example, observation in a living body in a medical field. In recent years, a capsule endoscope has been proposed that is swallowed by a subject and introduced into a body and picks up images while moving in the body according to a peristaltic movement. Image data picked up by the capsule endoscope in the body while the capsule endoscope moves in a body cavity is sequentially transmitted to an outside as image communication and accumulated in a memory provided in a receiver on an outside. After swallowing the capsule endoscope, the subject can act freely by carrying the receiver.

The capsule endoscope obtains driving power from a battery or the like built in a housing. Since the capsule endoscope has structure in which an inner circuit and the like are sealed in the housing, an operator cannot operate a switch or the like disposed on an outer surface of the housing to start and stop the capsule endoscope. Therefore, for example, Japanese Patent Application Laid-Open Publication No. 2009-89907 discloses a living body observation system including a magnetic field generating apparatus that generates a magnetic field and a capsule endoscope that controls, with a toggle operation, ON and OFF of supply of driving power according to a control signal by a magnetic field from an outside.

However, in the above living body observation system, the magnetic field generated by the magnetic field generating apparatus may leak into a space other than a space in which the capsule endoscope is disposed. There is a concern that this leakage magnetic field adversely affects other surrounding apparatuses.

Document EP 1 967 127 A1 describes a storage device for a medical device, wherein a container case contains a capsule endoscope which incorporates a magnet. A magnetic body is disposed so as to guide, from one magnetic pole to the other magnetic pole, a magnetic field which is generated from the magnet of the capsule endoscope that is accommodated between and positioned by a first hold portion of an outer case and a second hold portion of an inner case which position the capsule endoscope. The start control of the capsule is done by an infrared sensitive switch.

US 2007/129602 A1 discloses the activation of a capsule endoscope with an RF sensitive switch and an external RF generator.

It is an object of the present invention to provide a control signal transmitting apparatus in which a generated electromagnetic field is unlikely to adversely affect other surrounding apparatuses.

The object is solved by the features of claim 1. The dependent claims are directed to preferred embodiments of the invention.

### Brief Description of the Drawings

Fig. 1 is a perspective view showing an external appearance of a magnetic field generating apparatus according to a first embodiment;
Fig. 2 is a configuration diagram showing a configuration of a capsule endoscope controlled by the magnetic field generating apparatus according to the first embodiment;
Fig. 3 is a configuration diagram showing a circuit configuration of the capsule endoscope controlled by the magnetic field generating apparatus according to the first embodiment;
Fig. 4 is a configuration diagram showing a circuit configuration of the magnetic field generating apparatus according to the first embodiment;
Fig. 5 is a diagram for explaining cross sectional structure of the magnetic field generating apparatus according to the first embodiment;
Fig. 6 is a diagram for explaining cross sectional structure of a magnetic field generating apparatus according to a modification 1 of the first embodiment;
Fig. 7 is a diagram for explaining cross sectional structure of a magnetic field generating apparatus according to a modification 2 of the first embodiment;
Fig. 8 is a diagram for explaining cross sectional structure of a magnetic field generating apparatus according to a modification 3 of the first embodiment;
Fig. 9 is a diagram for explaining cross sectional structure of a magnetic field generating apparatus according to a modification 4 of the first embodiment;
Fig. 10 is a diagram for explaining cross sectional structure of a magnetic field generating apparatus according to a modification 5 of the first embodiment;
Fig. 11 is a perspective view showing an external appearance of a magnetic field generating apparatus according to a second embodiment;
Fig. 12 is a diagram for explaining cross sectional structure of a magnetic field generating apparatus according to a third embodiment;
Fig. 13 is a diagram for explaining cross sectional structure of a magnetic field generating apparatus according to a fourth embodiment;
Fig. 14 is a top view for explaining structure of the magnetic field generating apparatus according to the fourth embodiment;
Fig. 15 is a diagram for explaining cross sectional structure of a magnetic field generating apparatus according to a modification of the fourth embodiment;
Fig. 16 is a top view for explaining structure of the magnetic field generating apparatus according to the modification of the fourth embodiment;
Fig. 17 is a diagram for explaining cross sectional structure of a magnetic field generating apparatus according to a fifth embodiment; and
Fig. 18 is a diagram for explaining cross sectional structure of a magnetic field generating apparatus according to a sixth embodiment.

### Best Mode for Carrying Out the Invention

### First Embodiment

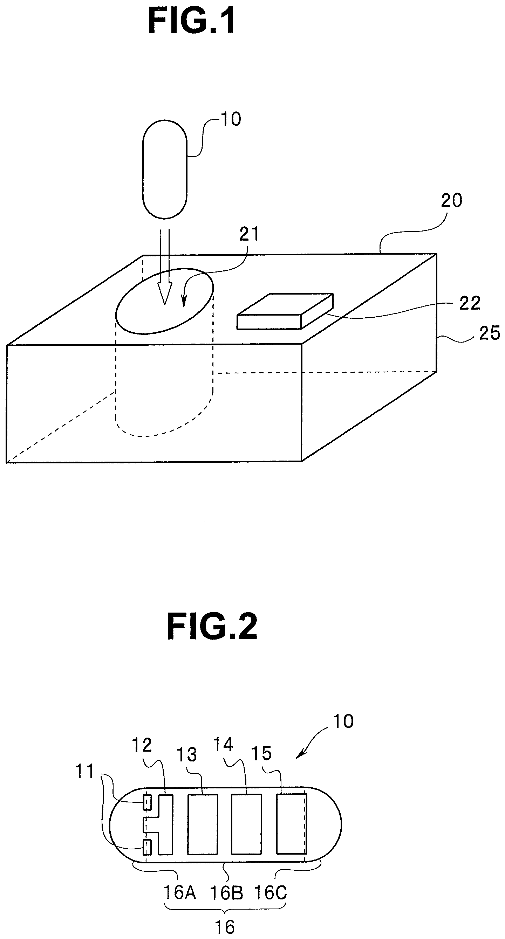

A magnetic field generating apparatus 20, which is a control signal transmitting apparatus, according to a first embodiment of the present invention is explained below with reference to Figs. 1 to 5. As shown in Fig. 1, a living body observation system according to the present embodiment includes a capsule endoscope 10 and the magnetic field generating apparatus 20. The capsule endoscope 10 can be stored in a storing section 21, which is an arrangement section, disposed in a housing 25 of the magnetic field generating apparatus 20. The capsule endoscope 10 is a living body information acquiring apparatus that can be introduced into a digestive organ tube cavity, which is an inside of a subject. The capsule endoscope 10 starts upon receiving a control signal by an electromagnetic field from the magnetic field generating apparatus 20 immediately before the subject swallows the capsule endoscope 10. The storing section 21 is a recessed section disposed in the housing 25, in other words, a wall section having a space inside. In the magnetic field generating apparatus 20, the capsule endoscope 10 is stored in the storing section 21 with a longitudinal direction set in a vertical direction. In the following explanation, an electromagnetic field is also referred to as magnetic field or electromagnetic wave.

The magnetic field generating apparatus 20 transmits, according to pressing operation of a switch 22 by an operator, a control signal by an electromagnetic field to the capsule endoscope 10 stored in the storing section 21. As explained later, the capsule endoscope 10 performs control of start and stop according to the control signal. In other words, the operator can perform, by using the magnetic field generating apparatus 20, operation of start and stop of the capsule endoscope 10 having structure in which an inner circuit and the like are sealed in the housing.

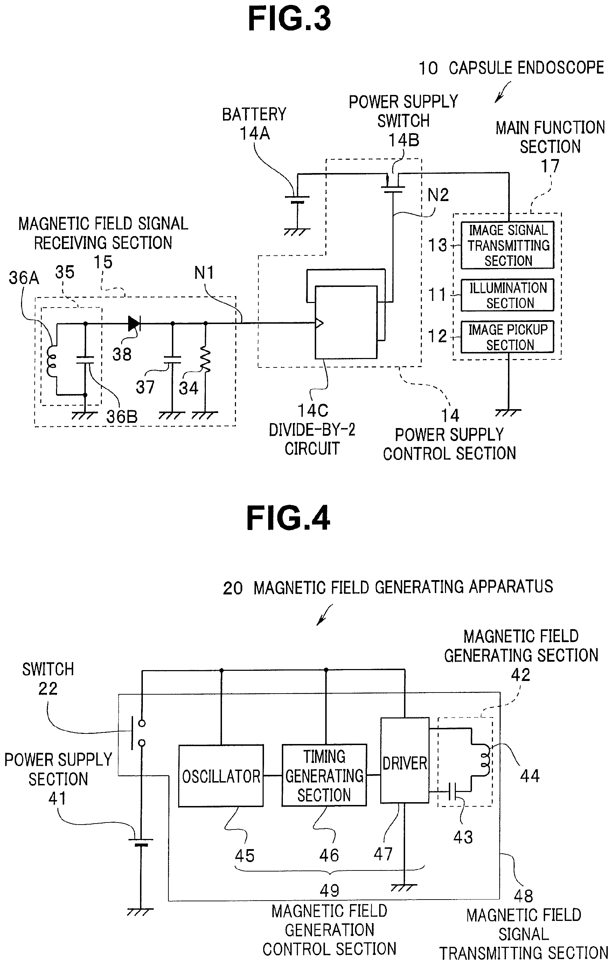

Next, the capsule endoscope 10 is explained using Figs. 2 and 3. As shown in Fig. 2, the capsule endoscope 10 includes an illumination section 11, an image pickup section 12, which is an information acquiring section, an image signal transmitting section 13, a power supply control section (hereinafter also referred to as "control section") 14, and a magnetic field signal receiving section 15, which are sealed inside of a housing 16. The housing 16 has an elongated capsule shape. An end 16A on the image pickup section 12 side has a dome shape formed of a transparent material. A cylindrical section 16B in a center and an end 16C of the dome shape on an opposite side are formed of a light blocking material. A capsule endoscope that has image pickup devices at both ends, which are formed of a transparent material, may be used.

Next, a circuit configuration of the capsule endoscope 10 is explained using FIG. 3. As shown in Fig. 3, a main function section 17 is configured by the illumination section 11, the image pickup section 12, and the image signal transmitting section 13.

The main function section 17 acquires an image in a body of a subject and transmits the image as an image signal. A battery 14A of the control section 14 is a power supply section that supplies electric power used for driving of the main function section 17. The magnetic field signal receiving section 15 receives a control signal by an AC magnetic field generated by the magnetic field generating apparatus 20, rectifies the control signal, and outputs the control signal as a direct current. The power supply control section 14 includes a divide-by-2 circuit 14C and a switch 14B. The power supply control section 14 controls ON and OFF of supply of electric power from the battery 14A to the main function section 17 on the basis of an electric signal from the magnetic field signal receiving section 15.

The illumination section 11 of the main function section 17 of the capsule endoscope 10 includes an LED (not shown) for irradiating an image pickup area when an inside of a body of a subject is photographed and an LED driving circuit (not shown) that controls a driving state of the LED. The image pickup section 12 includes a CCD (not shown) that performs image pickup of the area irradiated by the LED and a signal processing circuit (not shown) that processes an image signal outputted from the CCD into image data of a desired form. The image signal transmitting section 13 includes an image signal transmitting unit (not shown) that modulates the image data picked up by the CCD and processed and generates an image signal and a transmitting antenna section (not shown) that actually transmits the image signal outputted from the image signal transmitting unit. The LED is illustrated as illumination means. However, the illumination means is not limited to the LED as long as the illumination means can irradiate the image pickup area. The CCD is illustrated as image pickup means. However, the image pickup means is not limited to the CCD as long as the image pickup means can perform image pickup. The image pickup means may be, for example, a CMOS sensor.

The magnetic field signal receiving section 15 includes a receiving antenna section 35, a diode 38 that configures a rectifying circuit, a smoothing capacitor 37, and a resistor 34. The receiving antenna section 35 is a resonant circuit including a secondary side coil 36A and a secondary side capacitor 36B. The receiving antenna section 35 is adjusted to resonate with a frequency of a control signal. The secondary side coil 36A is, for example, a coil wound around in a circumferential direction of the housing 16. The secondary side coil 36A efficiently receives an electromagnetic field parallel to a longitudinal direction of the elongated housing 16.

A control signal by an AC magnetic field from an outside generates an alternating current in the secondary side coil 36A according to an electromagnetic induction action. The alternating current by the control signal received by the receiving antenna section 35 is rectified by the diode 38, smoothed by the smoothing capacitor 37, and transmitted to the divide-by-2 circuit 14C of the control section 14 as an electric signal of a direct current at a reception voltage level. In other words, since the capsule endoscope 10 magneto-electrically converts the received control signal and outputs the electric signal of the direct current, a power supply for the magnetic field signal receiving section 15 to detect a magnetic field is unnecessary.

The divide-by-2 circuit 14C includes a D type flip-flop circuit. The divide-by-2 circuit 14C outputs a signal obtained by dividing an inputted electric signal by 2 to the switch 14B. The switch 14B includes a P channel type FET, a source of which is connected to the battery 14A, a gate of which is connected to an output of the divide-by-2 circuit 14C, and a drain of which is connected to circuits of the main function section 17.

When the magnetic field signal receiving section 15 detects (receives) an AC magnetic field, an outputted electric signal (a node N1) changes to a high level of a reception voltage level. When the reception voltage level exceeds a threshold of the divide-by-2 circuit 14C, an output (a node N2) of the divide-by-2 circuit 14C changes to a ground voltage level. The switch 14B changes to an ON state. The main function section 17 of the capsule endoscope 10 starts an operation.

When an output signal of the divide-by-2 circuit 14C is at a power supply voltage level, the switch 14B is off. The electric power to the main function section 17 is not supplied. Conversely, when the output signal of the divide-by-2 circuit 14C is at a ground voltage level, the switch 14B is turned on. The electric power to the main function section 17 is supplied.

As explained above, the switch 14B performs, according to an operation of the magnetic field signal receiving section 15, a toggle operation in which ON and OFF states are switched. Specifically, the switch 14B changes from the OFF state to the ON state or from the ON state to the OFF state according to an operation of detecting a magnetic field once by the magnetic field signal receiving section 15. In other words, the divide-by-2 circuit 14C functions as a state holding section for the switch 14B. The D type flip-flop circuit of the divide-by-2 circuit 14C may be another circuit as long as the circuit can divide an input signal by 2. The D type flip-flop may be a T type flip-flop circuit or the like.

Next, a circuit configuration of the magnetic field generating apparatus 20, which is the control signal transmitting apparatus, is explained with reference to Fig. 4. As shown in Fig. 4, the magnetic field generating apparatus 20 includes a magnetic field generating section 42, which is an electromagnetic field generating section that generates a control signal by an AC magnetic field, a magnetic field generation control section 49 for driving the magnetic field generating section 42, a power supply section 41, and a switch 22 that inputs and cuts off electric power from the power supply section 41. The magnetic field generating section 42 configures a resonant circuit including a primary side coil (hereinafter simply referred to as "coil") 44 and a primary side capacitor 43. The magnetic field generation control section 49 includes an oscillator 45, a timing generating section 46, and a driver 47 that drives the magnetic field generating section 42.

The switch 22 is a switch operated by the operator and is, for example, a press button switch. The timing generating section 46 performs processing for, for example, setting a signal from the oscillator 45 to a desired frequency and transmits the signal having the frequency to the driver 47. The driver 47 drives the magnetic field generating section 42 according to the inputted signal. The primary side coil 44 generates an AC magnetic field having a predetermined frequency corresponding to the signal. The AC magnetic field functions as a stop signal for controlling start or stop of the capsule endoscope 10.

A frequency of the AC magnetic field generated by the magnetic field generating apparatus 20 as the control signal is desirably a frequency standardized as a frequency used by an RFID tag. This is not only because of a legal problem of electromagnetic wave emission but also because the magnetic field signal transmitting section 48 of the magnetic field generating apparatus 20, the magnetic field signal receiving section 15 of the capsule endoscope 10, and the like can be configured by inexpensive and highly reliable general-purpose electronic components.

As the frequency of the AC magnetic field, 13.56 MHz internationally standardized as an RFID tag frequency in ISO10536/14443/15693 of the International Standardization Organization is particularly desirable. Since the capsule endoscope 10 can use the electromagnetic wave of the control signal as energy by magneto-electrically converting the electromagnetic wave, the electric power of the built-in battery 14A is not consumed when the main function section 17 is stopped. Therefore, even if days elapse after manufacturing, in-vivo observable time can be secured.

However, the RFID tag is used for a large number of applications such as physical distribution management. Therefore, in the magnetic field generating apparatus 20 that generates an electromagnetic field having the frequency used by the RFID tag, in particular, it is important to reduce a leakage electromagnetic field.

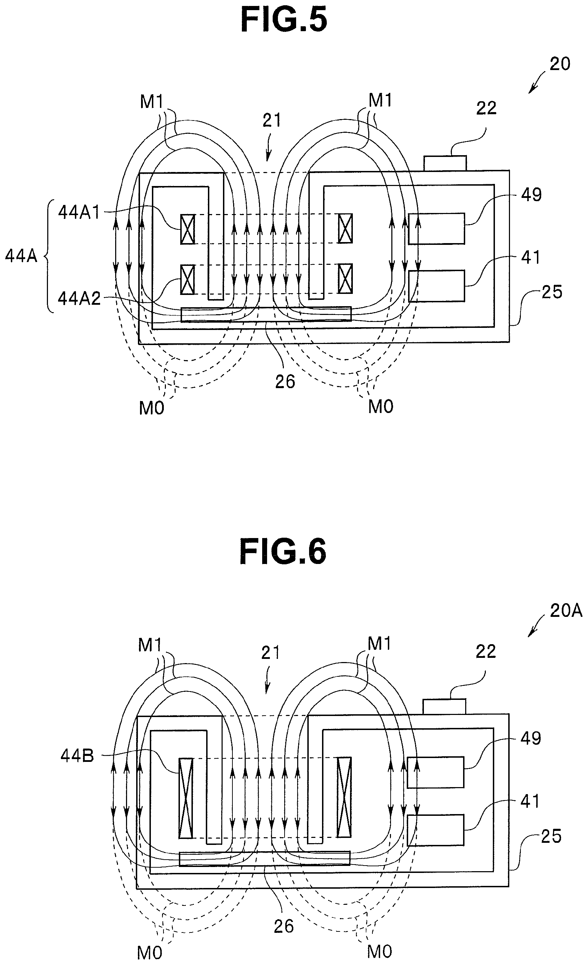

Next, a cross sectional structure of the magnetic field generating apparatus 20, which is the control signal transmitting apparatus, according to the present embodiment is explained with reference to Fig. 5. As shown in Fig. 5, a coil 44A is a Helmholtz coil in which a coil 44A1 and a coil 44A2 are arranged to be opposed to each other. In the storing section 21, the coil 44A generates an AC magnetic field in the vertical direction and a vertical counter direction. Therefore, the magnetic field generating apparatus 20 can efficiently apply a magnetic field to the secondary side coil 36A of the capsule endoscope 10 stored in the storing section 21 with the longitudinal direction of the housing 16 set in the vertical direction.

The magnetic field generating apparatus 20 includes, on a bottom of the housing 25, a shield section 26 that shields a leakage magnetic field leaking to an area other than the storing section 21 in the magnetic field generated by the coil 44A. The shield section 26 is formed of, for example, a high permeability soft magnetic material such as Permalloy or a conductor such as copper or aluminum. The shield section 26 formed of high permeability soft magnetic material confines a leakage magnetic field having a relatively low frequency inside of the shield section 26. The shield section 26 formed of the conductor reflects and absorbs a leakage electromagnetic wave having a relatively high frequency. It is possible to shield a magnetic field and an electromagnetic wave by using a soft magnetic material having conductivity in the shield section 26.

The shield section 26 may be an electromagnetic steel plate, an amorphous alloy, a metal mesh, conductive polymer, or the like, may be formed by plating, vacuum evaporation, metal thermal spraying, application of paint containing a conductive filler, sticking of a conductive tape, or the like, and may be an multi-layer film of a conductor layer and an insulating layer. The shield section 26 may be configured by forming plural shield materials in a laminated structure. Further, a shape of the shield section 26 viewed from above is not limited to a circular shape or a rectangular shape and may be a polygonal shape or the like.

Specifically, in Fig. 5, when the shield section 26 is absent, a magnetic field M0 (a broken line) leaks from the bottom of the housing 25. However, in the magnetic field generating apparatus 20, a leakage electromagnetic field of a magnetic field M1 (a solid line) leaking to the area other than the storing section 21 is shielded by the shield section 26.

In the magnetic field generating apparatus 20, which is the control signal transmitting apparatus, according to the present embodiment, since the leakage electromagnetic field leaking to the area other than the storing section 21 is shielded, a generated magnetic field is unlikely to adversely affect other surrounding apparatuses.

### Modifications of the first embodiment

Magnetic field generating apparatuses 20A and 20E according to modifications of the first embodiment of the present invention are explained below with reference to Figs. 6 to 10. Since the magnetic field generating apparatuses 20A to 20E according to the modifications are similar to the magnetic field generating apparatus 20 according to the first embodiment, components having the same functions are denoted by the same reference numerals and signs and explanation of the components is omitted.

As shown in Fig. 6, a coil 44B of the magnetic field generating apparatus 20A according to a modification 1 of the first embodiment is a solenoid type coil. The storing section 21 is disposed inside of a winding. Like the magnetic field generating apparatus 20, the magnetic field generating apparatus 20A can efficiently apply a magnetic field to the secondary side coil 36A of the capsule endoscope 10 stored in the storing section 21.

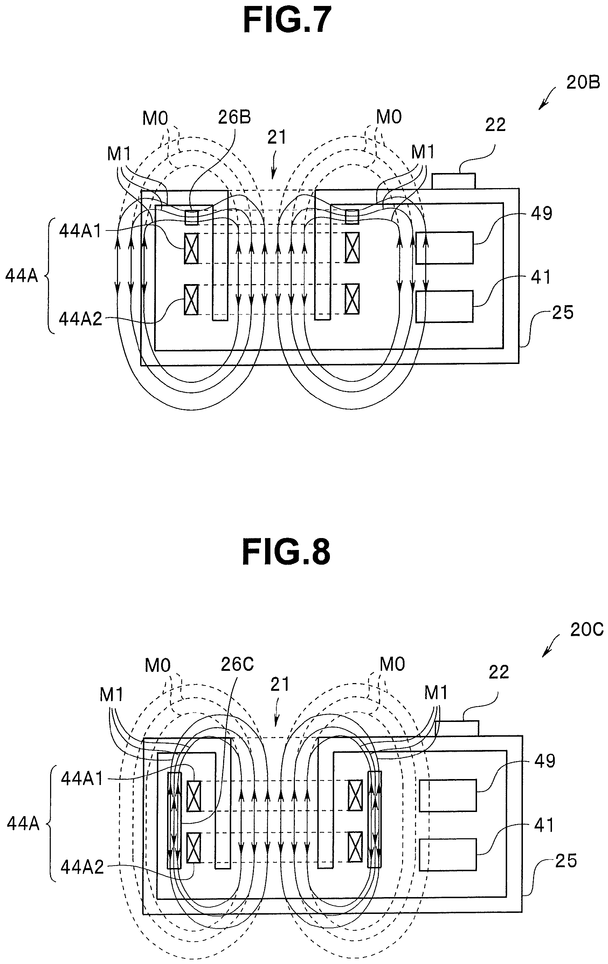

As shown in Fig. 7, a shield section 26B of the magnetic field generating apparatus 20B according to a modification 2 of the first embodiment has a doughnut shape disposed above the coil 44A. The magnetic field generating apparatus 20B can shield, in particular, a leakage magnetic field leaking to an upper surface side of the housing 25.

As shown in Fig. 8, a shield section 26C of a magnetic field generating apparatus 20C according to a modification 3 of the first embodiment has a cylindrical shape disposed on an outer circumferential section of the coil 44A. The magnetic field generating apparatus 20C can shield, in particular, a leakage magnetic field leaking to a side surface side of the housing 25.

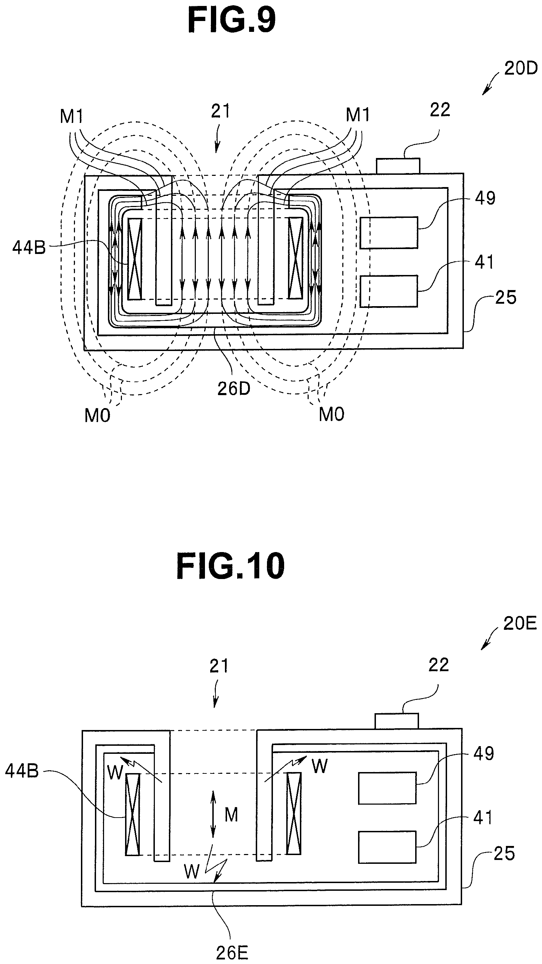

As shown in Fig. 9, a shield section 26D of a magnetic field generating apparatus 20D according to a modification 4 of the first embodiment has a bottomed cylindrical shape disposed to surround the coil 44B excluding a center portion of an upper surface. The magnetic field generating apparatus 20D can shield leakage magnetic fields leaking in respective directions of the housing 25.

As shown in Fig. 10, a shield section 26E of a magnetic field generating apparatus 20E according to a modification 5 of the first embodiment is formed over an entire inner wall surface of the housing 25. The shield section 26E may be stuck to a housing inner surface by machining a copper plate or may be directly formed as a film on an inner surface of the housing 25 by a plating method or the like. The magnetic field generating apparatus 20D can shield electromagnetic waves W leaking in respective directions from the housing 25. A material forming the housing 25 may be a material having a shield effect same as a shield effect of the shield section.

As explained above, in all the magnetic field generating apparatuses 20A to 20E according to the modifications, as in the magnetic field generating apparatus 20 according to the first embodiment, a leakage electromagnetic field leaking to the area other than the storing section 21 is shielded. Therefore, a generated magnetic field is unlikely to adversely affect other surrounding apparatuses. In all the modifications, a shape of the shield sections viewed from above is not limited to a circular shape, a rectangular shape, and the like and may be a polygonal shape.

### Second Embodiment

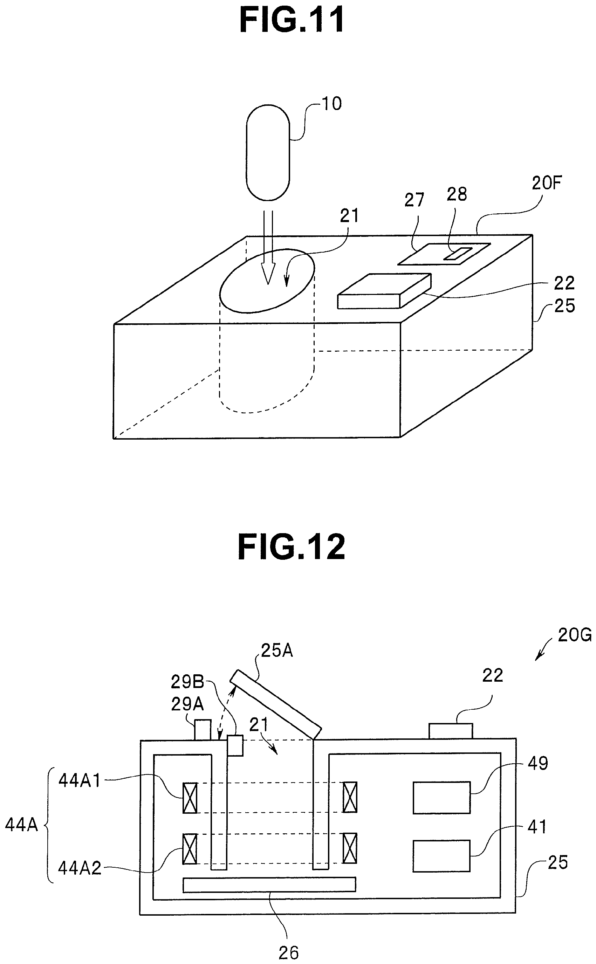

Next, a magnetic field generating apparatus 20F according to a second embodiment of the present invention is explained with reference to Fig. 11. Since the magnetic field generating apparatus 20F according to the present embodiment is similar to the magnetic field generating apparatus 20 according to the first embodiment, components having the same functions are denoted by the same reference numerals and signs and explanation of the components is omitted.

Since an operator cannot sense a magnetic field generated from a magnetic field generating apparatus, a magnetic field generated by mistake is likely to adversely affect other surrounding apparatuses. On the other hand, the magnetic field generating apparatus 20F according to the present embodiment includes a notifying section that notifies the operator of generation of a magnetic field.

Specifically, as shown in Fig. 11, a display section 27 including an LED 28 is disposed as the notifying section in an upper surface portion of the housing 25 of the magnetic field generating apparatus 20F. The LED 28 of the display section 27 is in a lit-out state when switch operation is not performed and a magnetic field is not generated. The LED 28 changes to a lit state when switch operation is performed and a magnetic field is generated. When a generation time of the magnetic field is so short that it is difficult for the operator to recognize the magnetic field, the lit state may be continued for a fixed period after the generation of the magnetic field.

A display method of the display section 27 is not limited to light and light-out display as long as the operator can recognize presence or absence of generation of a magnetic field. Blinking display, a change of a display color, or display by a character string using a liquid crystal display or the like may be performed.

Rather than changing a state of the display section 27 in association with the switch operation, a detecting section that detects intensity of an actually generated magnetic field may be provided to change a display state of the display section 27 stepwise on the basis of the detected magnetic field intensity. Since an electric current flowing to the magnetic field generating section 42 is in a proportional relation with the magnetic field intensity, the electric current may be measured to change the display state of the display section 27 stepwise on the basis of the measured electric current.

Further, the notifying section may be a sound generating section such as a speaker or a buzzer. The sound generating section notifies the operator of generation of a magnetic field with generation of sound, a level of sound, a tone, length of a sound generation time, voice, or the like. The notifying section may be a vibrating section using a vibration motor or the like. Moreover, the notifying section may be a combination of the display section 27, the sound generating section, and the vibrating section.

Since the magnetic field generating apparatus 20F notifies the operator of generation of a magnetic field with the notifying section, it is possible to prevent generation of an unnecessary magnetic field from adversely affecting other surrounding apparatuses.

### Third Embodiment

Next, a magnetic field generating apparatus 20G according to a third embodiment of the present invention is explained with reference to Fig. 12. Since the magnetic field generating apparatus 20G according to the present embodiment is similar to the magnetic field generating apparatus 20 according to the first embodiment, components having the same functions are denoted by the same reference numerals and signs and explanation of the components is omitted.

When a magnetic field generating apparatus includes the storing section 21, which is the recessed section, as the arrangement section in which the capsule endoscope 10 is arranged, it is likely that foreign matters are mixed in the storing section 21. On the other hand, the magnetic field generating apparatus 20G according to the present embodiment includes an openable and closable cover section 25A that covers an opening of the storing section 21. Further, the magnetic field generating apparatus 20G includes an opening and closing detecting section 29A that detects open and closed states of the cover section 25A and a lock section 29B that limits opening and closing of the cover section 25A.

Since the magnetic field generating apparatus 20G includes the cover section 25A, it is possible to prevent mixing of foreign matters, for example, foreign matters formed of a magnetic body attracted by a magnetic field in the storing section 21. Therefore, the magnetic field generating apparatus 20G can perform a stable operation. Further, the magnetic field generating apparatus 20G includes the opening and closing detecting section 29A and, when the opening and closing detecting section 29A detects that the cover section 25A is in an open state, the magnetic field generation control section 49 controls the magnetic field generating section 42 not to generate a magnetic field. Therefore, the magnetic field generating apparatus 20G can prevent generation of an unnecessary magnetic field.

The magnetic field generating apparatus 20G including the lock section 29B can also control the cover section 25A not to open while a magnetic field is generated. Therefore, the magnetic field generating apparatus 20G can prevent careless pull-out of the capsule endoscope 10 and obtain a stable operation state.

The cover section 25A is desirably formed of a material having an electromagnetic field shield effect or formed by a combination with a member having the electromagnetic field shield effect. The cover section 25A having the electromagnetic field shield effect can reduce a leakage magnetic field leaking to an upper part of the storing section 21.

### Fourth Embodiment

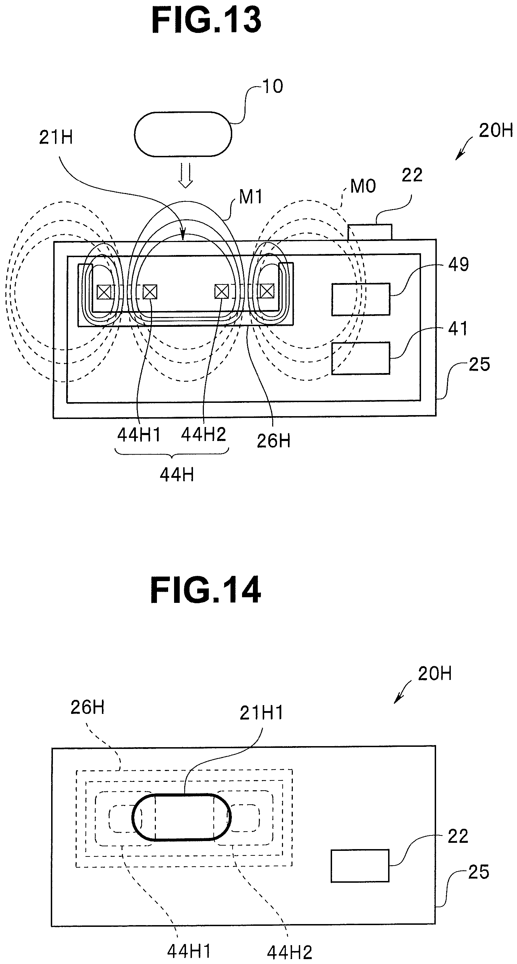

Next, a magnetic field generating apparatus 20H according to a fourth embodiment is explained with reference to Figs. 13 and 14. Since the magnetic field generating apparatus 20H according to the present embodiment is similar to the magnetic field generating apparatus 20 according to the first embodiment, components having the same functions are denoted by the same reference numerals and signs and explanation of the components is omitted.

As shown in Figs. 13 and 14, an arrangement section of the magnetic field generating apparatus 20H is a flat section 21H on a surface of the upper surface portion of the housing 25. A coil 44H including two coils 44H1 and 44H2 is arranged such that a generated magnetic field is efficiently applied to the flat section 21H. A shield section 26H has a box shape that surrounds side surfaces and a bottom surface of the coil 44H. The shield section 26H shields a leakage electromagnetic field leaking to an area other than the flat section 21H.

Since the flat section 21H is formed of a part of the housing 25, it is not easy to specify a location of the flat section 21H from an outside. Therefore, as shown in Fig. 14, a mark 21H1, which imitates an outer shape of the capsule endoscope 10, indicating a position of the flat section 21 H is drawn on the upper surface portion of the housing 25.

In the magnetic field generating apparatus 20H, the arrangement section in which the capsule endoscope 10 is arranged is the flat section 21H. Therefore, even if foreign matters or the like adhere to the arrangement section, it is possible to easily remove the foreign matters and it is easy to keep an aseptic state by disinfection treatment.

### Modification of the fourth embodiment

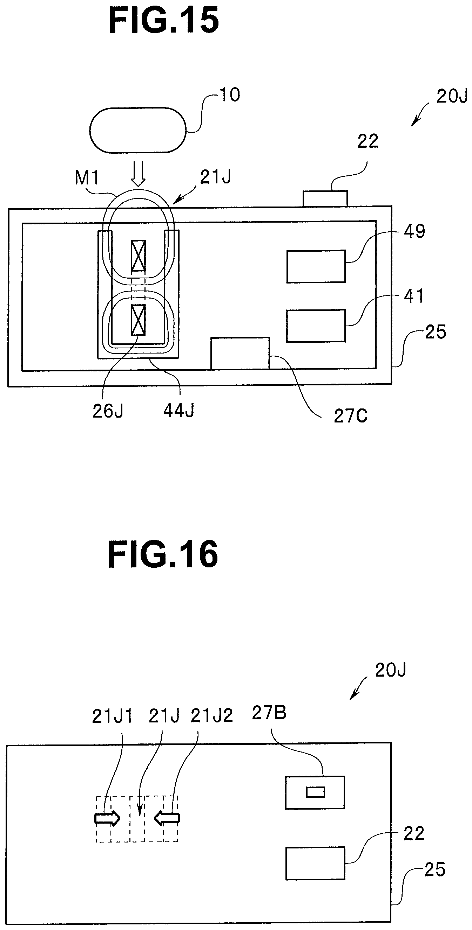

Next, a magnetic field generating apparatus 20J according to a modification of the fourth embodiment of the present invention is explained with reference to Figs. 15 and 16. Since the magnetic field generating apparatus 20J according to the present modification is similar to the magnetic field generating apparatus 20H according to the fourth embodiment, components having the same functions are denoted by the same reference numerals and signs and explanation of the components is omitted.

An arrangement section of the magnetic field generating apparatus 20J in which the capsule endoscope 10 is arranged is a flat section 21J as in the magnetic field generating apparatus 20H. However, a coil 44J is one wound coil. A shield section 26J has a box shape that surrounds side surfaces and a bottom surface of the coil 44J. Distinguishing marks 21J1 and 21J2 of arrows indicating a position of the flat section 21J are drawn on the surface of the housing 25.

The magnetic field generating apparatus 20J according to the present modification has effects same as those of the magnetic field generating apparatus 20H.

Further, the magnetic field generating apparatus 20J includes, as a notifying section that notifies generation of a magnetic field, a speaker 27B, which is a sound generating section, on the upper surface of the housing 25 and a vibrating motor 27C, which is a vibrating section, inside of the housing 25. Therefore, the magnetic field generating apparatus 20J can surely notify an operator of generation of a magnetic field.

### Fifth Embodiment

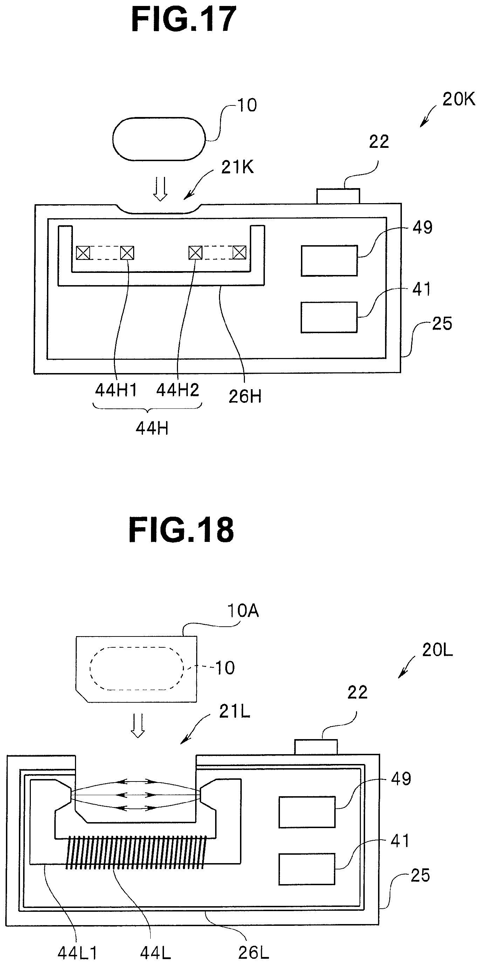

Next, a magnetic field generating apparatus 20K according to a fifth embodiment of the present invention is explained with reference to Fig. 17. Since the magnetic field generating apparatus 20K according to the present embodiment is similar to the magnetic field generating apparatus 20H according to the fourth embodiment, components having the same functions are denoted by the same reference numerals and signs and explanation of the components is omitted.

As shown in FIG. 17, an arrangement section of the magnetic field generating apparatus 20K is a storing section 21K in the upper surface portion of the housing 25. The storing section 21 K has a shape matched to an outer diameter shape of the capsule endoscope 10. In other words, the capsule endoscope 10 can be fit in the storing section 21K. A magnetic field generated by the coil 44H has a distribution. A range of high magnetic field intensity is not large. However, in the magnetic field generating apparatus 20K, an operator can surely fix the capsule endoscope 10 in a predetermined position where magnetic field intensity is high.

The storing section 21K is a shallow recessed section formed of a curved surface in order to store the capsule endoscope 10 in a lateral direction, so that even if foreign matters are mixed in, it is possible to easily remove the foreign matters. A shape of the storing section 21K only has to have a size enough for fitting the capsule endoscope 10 in a hollow of the recessed section so as not to easily shift.

In addition to the effects of the magnetic field generating apparatus 20 and the like, the magnetic field generating apparatus 20K can more surely apply a magnetic field to the capsule endoscope 10. Also, it is easy to maintain cleanness of the storing section 21K.

### Sixth Embodiment

Next, a magnetic field generating apparatus 20L according to a sixth embodiment of the present invention is explained with reference to Fig. 18. Since the magnetic field generating apparatus 20L according to the present embodiment is similar to the magnetic field generating apparatus 20H according to a fourth embodiment, components having the same functions are denoted by the same reference numerals and signs and explanation of the components is omitted.

As shown in Fig. 18, in the magnetic field generating apparatus 20L, the capsule endoscope 10 is arranged in a storing section 21 L, which is an arrangement section, in a state in which the capsule endoscope 10 is sealed in a storage container 10A. The capsule endoscope 10 is started in the sealed state. An aseptic state is maintained until the storage container 10A is opened immediately before the capsule endoscope 10 is introduced into a body.

The storage container 10A and the storing section 21L have shapes that fit with each other. Therefore, an operator can surely fix the capsule endoscope 10 in a predetermined position where magnetic field intensity is high.

Further, as shown in Fig. 18, a coil 44L of the magnetic field generating apparatus 20L is wound around a magnetic yoke 44L1 formed of Permalloy, which is a soft magnetic body. Therefore, a leakage magnetic field is little. Further, ends of the magnetic yoke 44L1 arranged to be opposed to each other across the storing section 21L have a tapered shape. Therefore, a magnetic field can be efficiently introduced into the storing section 21 L. As in the magnetic field generating apparatus 20E according to the modification 5 of the first embodiment, the inner surface of the housing 25 is covered with a shield section 26L formed of Permalloy, which is a soft magnetic body having conductivity.

In addition to the effects of the magnetic field generating apparatus 20 and the like, the magnetic field generating apparatus 20L can more surely apply a magnetic field to the capsule endoscope 10.

In the above explanation, the capsule endoscope is explained as the example of the living body information acquiring apparatus. However, the control signal transmitting apparatus according to the present invention can be applied to various living body information acquiring apparatuses of a capsule such as a capsule medical apparatus for sampling digestive fluid, a capsule temperature sensor of a swallow type, or a capsule pH sensor of a swallow type.

The present invention is not limited to the embodiments explained above. Various changes, alterations, and the like are possible in a range within the scope of the claims.

## Claims

1. A control signal transmitting apparatus (20, 20A-20L) that transmits a control signal by an electromagnetic field to a living body information acquiring apparatus (10) that performs control of start and stop according to the control signal, comprising:
a housing (25) including an arrangement section (21, 21 H-21 L) in which the living body information acquiring apparatus (10) may be arranged;
an electromagnetic field generating section (42) that generates the electromagnetic field; and
a shield section (26, 26B-26E, 26H, 26J-26L) adapted to shield a leakage of the generated electromagnetic field leaking to an area other than the arrangement section (21, 21 H-21 L) in the electromagnetic field generated by the electromagnetic field generating section (42), wherein
the electromagnetic field is an AC magnetic field; and
the electromagnetic field generating section (42) and the shield section (26, 26B-26E, 26H, 26J-26L) are arranged in the housing (25).

2. The control signal transmitting apparatus (20, 20A-20L) according to claim 1, further comprising a notifying section (27, 27B, 27C) that notifies the generation of the electromagnetic field when the electromagnetic field generating section (42) is generating the electromagnetic field.

3. The control signal transmitting apparatus (20, 20A-20L) according to claim 2, wherein the notifying section (27, 27B, 27C) is a display section (27).

4. The control signal transmitting apparatus (20, 20A-20L) according to claim 2, wherein the notifying section (27, 27B, 27C) is a sound generating section (27B).

5. The control signal transmitting apparatus (20, 20A-20L) according to claim 2, wherein the notifying section (27, 27B, 27C) is a vibrating section (27C).

6. The control signal transmitting apparatus (20, 20A-20L) according to claim 1, wherein the arrangement section (21, 21 H, 21 K, 21 L) is a recessed section arranged in the housing (25), the living body information acquiring apparatus (10) being capable of being inserted into the recessed section.

7. The control signal transmitting apparatus (20G) according to claim 6, further comprising an openable and closable cover section (25A) that covers an opening of the recessed section.

8. The control signal transmitting apparatus (20G) according to claim 7, further comprising a lock section (29B) that limits opening and closing of the cover section.

9. The control signal transmitting apparatus (20G) according to claim 7, further comprising an opening and closing detecting section (29A) that detects open and closed states of the cover section, wherein
the electromagnetic field generating section (42) does not generate the electromagnetic field when the opening and closing detecting section (29A) detects that the cover section is in the open state.

10. The control signal transmitting apparatus (20K, 20L) according to claim 6, wherein the recessed section (21 K, 21 L) has a shape in which the living body information acquiring apparatus (10) or a storage container (10A) in which the living body information acquiring apparatus (10) is stored can be fit.

11. The control signal transmitting apparatus (20J) according to claim 1, wherein the arrangement section (21 J) is a flat section on a surface of the housing (25).

12. The control signal transmitting apparatus (20L) according to claim 1, wherein the electromagnetic field generating section (42) includes a wound coil section (44L) and a yoke section (44L1) that guides the electromagnetic field generated by the wound coil to the arrangement section (21, 21 H-21L).

13. The control signal transmitting apparatus (20, 20A-20L) according to claim 1, wherein a frequency of the control signal is a frequency used by an RFID tag.

14. The control signal transmitting apparatus (20, 20A-20L) according to claim 1, wherein the living body information acquiring apparatus (10) is a capsule endoscope.

## Patentansprüche

1. Steuersignalübertragungsvorrichtung (20, 20A - 20L), die ein Steuersignal durch ein elektromagnetisches Feld an eine Vorrichtung (10) zum Erlangen von Lebendkörperinformationen überträgt, die eine Steuerung eines Startens und Stoppens entsprechend dem Steuersignal durchführt, wobei die Vorrichtung aufweist:
ein Gehäuse (25), das einen Anordnungsabschnitt (21, 21H - 21 L) enthält, in dem die Vorrichtung (10) zur Erlangung von Lebendkörperinformationen angeordnet werden kann;
einen Elektromagnetfelderzeugungsabschnitt (42), der das elektromagnetische Feld erzeugt; und
einen Abschirmungsabschnitt (26, 26B - 26E, 26H, 26J - 26L), der ausgelegt ist, ein Austreten des erzeugten elektromagnetischen Feldes, das in einen Bereich austritt, der nicht der Anordnungsabschnitt (21, 21H - 21 L) ist, in dem elektromagnetischen Feld, das von dem Elektromagnetfelderzeugungsabschnitt (42) erzeugt wird, abzuschirmen, wobei
das elektromagnetische Feld ein AC-Magnetfeld ist; und
der Elektromagnetfelderzeugungsabschnitt (42) und der Abschirmungsabschnitt 26, 26B - 26E, 26H, 26J - 26L) in dem Gehäuse (25) angeordnet sind.

2. Steuersignalübertragungsvorrichtung (20, 20A - 20L) nach Anspruch 1, die außerdem einen Meldeabschnitt (27, 27B, 27C) aufweist, der die Erzeugung des elektromagnetischen Feldes meldet, wenn der Elektromagnetfelderzeugungsabschnitt (42) das elektromagnetische Feld erzeugt.

3. Steuersignalübertragungsvorrichtung (20, 20A - 20L) nach Anspruch 2, wobei der Meldeabschnitt (27, 27B, 27C) ein Anzeigeabschnitt (27) ist.

4. Steuersignalübertragungsvorrichtung (20, 20A - 20L) nach Anspruch 2, wobei der Meldeabschnitt (27, 27B, 27C) ein Tonerzeugungsabschnitt (27B) ist.

5. Steuersignalübertragungsvorrichtung (20, 20A - 20L) nach Anspruch 2, wobei der Meldeabschnitt (27, 27B, 27C) ein vibrierender Abschnitt (27C) ist.

6. Steuersignalübertragungsvorrichtung (20, 20A - 20L) nach Anspruch 1, wobei der Anordnungsabschnitt (21, 21 H, 21 K, 21 L) ein vertiefter Abschnitt ist, der in dem Gehäuse (25) angeordnet ist, wobei die Vorrichtung (10) zur Erlangung von Lebendkörperinformationen in der Lage ist, in den vertieften Abschnitt eingeführt zu werden.

7. Steuersignalübertragungsvorrichtung (20G) nach Anspruch 6, die außerdem einen zu öffnenden und zu schließenden Deckeiabschnitt (25A) aufweist, der eine Öffnung des vertieften Abschnitts bedeckt.

8. Steuersignalübertragungsvorrichtung (20G) nach Anspruch 7, die außerdem einen Verriegelungsabschnitt (29B) aufweist, der ein Öffnen und Schließen des Deckelabschnitts beschränkt.

9. Steuersignalübertragungsvorrichtung (20G) nach Anspruch 7, die außerdem einen Öffnungs- und Schließerfassungsabschnitt (29A) aufweist, der geöffnete und geschlossene Zustände des Deckelabschnitts erfasst, wobei
der Elektromagnetfelderzeugungsabschnitt (42) das elektromagnetische Feld nicht erzeugt, wenn der Öffnungs- und Schließerfassungsabschnitt (29A) erfasst, dass sich der Deckelabschnitt in dem geöffneten Zustand befindet.

10. Steuersignalübertragungsvorrichtung (20K, 20L) nach Anspruch 6, wobei der vertiefte Abschnitt (21 K, 21 L) eine Gestalt aufweist, in die die Vorrichtung (10) zum Erlangen von Lebendkörperinformationen oder ein Speicherbehälter (10A), in dem die Vorrichtung (10) zum Erlangen von Lebendkörperinformationen untergebracht wird, eingepasst werden kann.

11. Steuersignalübertragungsvorrichtung (20J) nach Anspruch 1, wobei der Anordnungsabschnitt (21 J) ein flacher Abschnitt auf einer Oberfläche des Gehäuses (25) ist.

12. Steuersignalübertragungsvorrichtung (20L) nach Anspruch 1, wobei der Elektromagnetfelderzeugungsabschnitt (42) einen gewickelten Spulenabschnitt (44L) und einen Jochabschnitt (44L1) enthält, der das elektromagnetische Feld, das von der gewickelten Spule erzeugt wird, zu dem Anordnungsabschnitt (21, 21H - 21 L) leitet.

13. Steuersignalübertragungsvorrichtung (20, 20A - 20L) nach Anspruch 1, wobei eine Frequenz des Steuersignals eine Frequenz ist, die von einem RFID-Etikett verwendet wird.

14. Steuersignalübertragungsvorrichtung (20, 20A - 20L) nach Anspruch 1, wobei die Vorrichtung (10) zum Erlangen von Lebendkörperinformationen ein Kapselendoskop ist.

## Revendications

1. Appareil de transmission de signal de commande (20, 20A à 20L) qui transmet un signal de commande par un champ électromagnétique à un appareil d'acquisition d'informations de corps vivant (10) qui réalise une commande de démarrage et d'arrêt selon le signal de commande, comprenant :
un logement (25) comportant une section d'agencement (21, 21H à 21L) dans laquelle l'appareil d'acquisition d'informations de corps vivant (10) peut être agencé ;
une section de génération de champ électromagnétique (42) qui génère le champ électromagnétique ; et
une section de protection (26, 26B à 26E, 26H, 26J à 26L) adaptée pour protéger une fuite du champ électromagnétique généré qui fuit vers une zone autre que la section d'agencement (21, 21H à 21L) dans le champ électromagnétique généré par la section de génération de champ électromagnétique (42), dans lequel
le champ électromagnétique est un champ magnétique à courant alternatif ; et
la section de génération de champ électromagnétique (42) et la section de protection (26, 26B à 26E, 26H, 26J à 26L) sont agencées dans le logement (25).

2. Appareil de transmission de signal de commande (20, 20A à 20L) selon la revendication 1, comprenant en outre une section de notification (27, 27B, 27C) qui notifie la génération du champ électromagnétique lorsque la section de génération de champ électromagnétique (42) génère le champ électromagnétique.

3. Appareil de transmission de signal de commande (20, 20A à 20L) selon la revendication 2, dans lequel la section de notification (27, 27B, 27C) est une section d'affichage (27).

4. Appareil de transmission de signal de commande (20, 20A à 20L) selon la revendication 2, dans lequel la section de notification (27, 27B, 27C) est une section de génération de son (27B).

5. Appareil de transmission de signal de commande (20, 20A à 20L) selon la revendication 2, dans lequel la section de notification (27, 27B, 27C) est une section de vibration (27C).

6. Appareil de transmission de signal de commande (20, 20A à 20L) selon la revendication 1, dans lequel la section d'agencement (21, 21H, 21K, 21L) est une section en retrait agencée dans le logement (25), l'appareil d'acquisition d'informations de corps vivant (10) étant capable d'être inséré dans la section en retrait.

7. Appareil de transmission de signal de commande (20G) selon la revendication 6, comprenant en outre une section de couvercle ouvrable et refermable (25A) qui couvre une ouverture de la section en retrait.

8. Appareil de transmission de signal de commande (20G) selon la revendication 7, comprenant en outre une section de verrou (29B) qui limite une ouverture et une fermeture de la section de couvercle.

9. Appareil de transmission de signal de commande (20G) selon la revendication 7, comprenant en outre une section de détection d'ouverture et de fermeture (29A) qui détecte des états ouverts et fermés de la section de couvercle, dans lequel
la section de génération de champ électromagnétique (42) ne génère pas le champ électromagnétique lorsque la section de détection d'ouverture et de fermeture (29A) détecte que la section de couvercle est dans l'état ouvert.

10. Appareil de transmission de signal de commande (20K, 20L) selon la revendication 6, dans lequel la section en retrait (21K, 21L) a une forme dans laquelle l'appareil d'acquisition d'informations de corps vivant (10) ou un contenant de stockage (10A) dans lequel l'appareil d'acquisition d'informations de corps vivant (10) est stocké peut être installé.

11. Appareil de transmission de signal de commande (20J) selon la revendication 1, dans lequel la section d'agencement (21J) est une section plate sur une surface du logement (25).

12. Appareil de transmission de signal de commande (20L) selon la revendication 1, dans lequel la section de génération de champ électromagnétique (42) comporte une section de bobine enroulée (44L) et une section de collier de bobinage (44L1) qui guide le champ électromagnétique généré par la bobine enroulée à la section d'agencement (21, 21H à 21L).

13. Appareil de transmission de signal de commande (20, 20A à 20L) selon la revendication 1, dans lequel une fréquence du signal de commande est une fréquence utilisée par une étiquette RFID.

14. Appareil de transmission de signal de commande (20, 20A à 20L) selon la revendication 1, dans lequel l'appareil d'acquisition d'informations de corps vivant (10) est un endoscope à capsule.
